# EUROPÄISCHE PATENTSCHRIFT

(11) **EP 0 697 394 B1**
(45) Veröffentlichungstag und Bekanntmachung des Hinweises auf die Patenterteilung: **14.01.1998**
(21) Anmeldenummer: 95111994.0
(22) Anmeldetag: 31.07.1995
(51) Int. Cl.: C07C 201/08

(54) **Verfahren zur Herstellung von Nitroaromaten**
Process for the preparation of nitroaromatics
Procédé de préparation de nitroaromatiques

(30) Priorität: 11.08.1994 DE 4428460
(43) Veröffentlichungstag der Anmeldung: 21.02.1996
(73) Patentinhaber: BAYER AG, 51368 Leverkusen (DE)
(72) Erfinder: Klingler, Uwe, D-41539 Dormagen (DE); Schieb, Thomas, Dr., D-51503 Rösrath (DE); Wiechers, Gerhard, Dr., D-51381 Leverkusen (DE); Zimmermann, Jürgen, Dr., Walnut Creek, CA 94598 (DE)

(56) Entgegenhaltungen:
- EP-A- 0 156 199
- EP-A- 0 597 361

## Beschreibung

Die Erfindung betrifft ein Verfahren zur kontinuierlichen Herstellung von Nitroaromaten durch Nitrierung von Aromaten mit Nitriersäure.

Nitroaromaten sind Zwischenprodukte für Kunststoffvorprodukte, Farbstoffe, Pflanzenschutzmittel, Pharmazeutika und Sprengstoffe. Nitroaromaten werden durch Umsetzung der aromatischen Ausgangsverbindung mit Salpetersäure gewonnen (Ullmanns Encyklopädie der technischen Chemie, 4. Aufl., Bd. 17, S. 383). Als Hilfsstoff wird u.a. Schwefelsäure verwendet. Bei der Nitrierung wird als Nebenprodukt Wasser gebildet, welches die Schwefelsäure verdünnt. Die so erhaltene, verdünnte Schwefelsäure muß aufkonzentriert werden, um sie erneut dem Prozeß in der Anfangskonzentration zuzuführen.

Die Aufkonzentrierung kann bei Normaldruck nach dem Pauling-Plinke-Verfahren durchgeführt werden (Bodenbrenner, von Plessen, Vollmüller, Dechema-Monogr. 86 (1980), 197). Hierbei wird die aufzukonzentrierende Säure über einen Dephlegmator in einen beheizten Kessel mit siedender konzentrierter Schwefelsäure gegeben.

Bei einem anderen bekannten Verfahren wird die Schwefelsäure im Vakuum bei reduziertem Druck ein- oder mehrstufig aufkonzentriert (Winnacker, Küchler, Chem. Technol., Bd. 2, Anorg. Technol. I, 4. Aufl., 1982, S. 70 bis 72).

Eine spezielle Ausführungsform dieses Vakuumverfahrens wird häufig im Anschluß von adiabatischen Nitrierverfahren durchgeführt. Hierbei wird die heiße, aufzukonzentrierende Schwefelsäure aus dem Reaktor kommend direkt ins Vakuum entspannt, wobei das Wasser über Kopf abgetrieben wird. Die aufkonzentrierte Säure fällt im Sumpf an. Ein solches Verfahren wird z.B. in EP-A-597 361 offenbart.

Allen diesen Aufkonzentrierungsverfahren ist gemeinsam, daß zusätzlich mit dem Wasser in der Schwefelsäure gelöste organische Verbindungen überdestillieren. Diese werden dann gemeinsam mit dem Wasser kondensiert. Hierbei ergibt sich das Problem, daß Organika, welche einen über dem Kondensationspunkt des Wasserdampfes liegenden Festpunkt aufweisen, in den festen Aggregatzustand übergehen und so die Wärmeübertragungsflächen der Kondensatoren belegen und diese unwirksam machen. Dieses ist beispielsweise der Fall bei der Aufkonzentrierung von Absäuren bei der Dinitrotoluolherstellung. Dort wird, um Wasser unter den vorgegebenen Druckbedingungen zu kondensieren, Kühlwasser mit vorzugsweise einer Temperatur von kleiner gleich 20°C verwendet. Das Dinitrotoluolisomerengemisch wird jedoch bereits bei einer Temperatur von ca. 55°C fest.

Aus der Literatur ist bekannt, daß sich ein Zuwachsen der Wärmeübertragungsflächen umgehen laßt, wenn getaktete Wärmeüberträger verwendet werden. Diese kommen jeweils wechselseitig zum Einsatz und werden in der Ruhephase gereinigt (z.B. abgeschmolzen). Problematisch ist hierbei jedoch weiterhin, daß sich die Wärmeübertragungsflächen während des Prozesses belegen und dadurch der Wärmedurchgang behindert wird. Nachteilig ist auch die hohe Taktfrequenz, mit der die Wärmeüberträger eingesetzt werden müssen.

Ein weiteres bekanntes Verfahren besteht darin, daß in die Brüden ein geeignetes Lösungsmittel eingesprüht wird, um dadurch die Organika in dem Lösungsmittel zu lösen und damit flüssig zu halten. Nachteilig hierbei ist, daß ein geeignetes Lösungsmittel oftmals nicht zur Verfügung steht oder aus sicherheitstechnischen Gründen oder aus Qualitätsgründen im Prozeß nicht toleriert werden kann. So wird z.B. in DE-A 23 09 719 bei der Aufkonzentrierung von gebrauchter Schwefelsäure aus der Dinitrotoluolherstellung Mononitrotoluol in den Brüdenstrom eingebracht, um das Auskristallisieren von Dinitrotoluol zu verhindern. Dies ist jedoch nur möglich, weil Dinitrotoluol hier nach dem zweistufigen Verfahren über Mononitrotoluol als Zwischenprodukt hergestellt wird und somit das eingesetzte Mononitrotoluol vor Ort zur Verfügung steht. Wird Dinitrotoluol wie z.B. in EP-A 0 066 202 einstufig hergestellt, so wird kein Mononitrotoluol als Zwischenprodukt erhalten, so daß ein Einsprühen in den Brüdenstrom nur mit zugekauftem Mononitrotoluol möglich ist.

Den zu kondensierenden Dampf unmittelbar mit dem Kühlmedium in Berührung zu bringen, die Brüden also z.B. durch Eindüsen von kalten Flüssigkeiten niederzuschlagen, ist ein seit langem bekanntes technisches Verfahren (Misch- oder Einspritzkondensator, siehe R.A. Vauck, H.A. Müller, Grundoperationen chemischer Verfahrenstechnik, 5. Aufl., VEB Leipzig 1962, S. 447). Problematisch ist diese Technik, wie bereits oben erwähnt, bei Anwesenheit organischer Verbindungen mit einem Festpunkt knapp oberhalb der Kondensationstemperaturen. Bedingt durch die hohe Erstarrungsgeschwindigkeit bilden sich amorphe, klebrige Niederschläge, die zu Verstopfungen in Rohrleitungen und Armaturen und zu Wandbelagbildungen in Wärmeüberträgern führen.

Aufgabe war es daher, ein Verfahren zur Verfügung zu stellen, welches eine effektive Kondensation der Brüden bei der Aufkonzentrierung von Schwefelsäure aus der Nitrierung gestattet, nicht zu einer Belegung der Wärmeübertragungsflächen führt und keine Lösungsmittel zum Lösen eventueller Niederschläge verwendet.

Diese Aufgabe wird durch das erfindungsgemäße Verfahren gelöst.

Gegenstand der Erfindung ist ein Verfahren zur kontinuierlichen Herstellung von Nitroaromaten durch Nitrierung von Aromaten, indem man den Aromaten in einem Reaktor unter Verwendung einer Nitriersäure, bestehend aus 80 bis 100 Gew.-% an anorganischen Bestandteilen, die sich im wesentlichen aus 60 bis 90 Gew.-% Schwefelsäure, 1 bis 20 Gew.-% Salpetersäure und mindestens 5 Gew.-% Wasser zusammensetzen, und bis zu 20 Gew.-% organischen Bestandteilen, die zu 70 bis 100 Gew.-% aus nitrierten Aromaten und zum Rest aus Nebenprodukten bestehen, unter Einstellung eines Molverhältnisses von Salpetersäure zu Aromat von 0,8 bis 2,5 : 1 in Abhängigkeit von der Nitrierung zur Reaktion bringt, das den Reaktor mit einer Temperatur von mindestens 80°C verlassende Reaktionsgemisch in eine obere Produktphase und eine untere Säurephase auftrennt, die Produktphase aufarbeitet und die mit Organika behaftete Säurephase durch Entspannungsverdampfung gegebenenfalls unter gleichzeitiger Wärmezufuhr, von mindestens 5 Gew.-% des Wassers befreit, welches dadurch gekennzeichnet ist, daß man nach der Entspannungsverdampfung die Brüden direkt in den Sprühstrahl eines Kühlmittels, vorzugsweise Wasser, im Vakuum einbringt, dabei die Brüden kondensiert, einen Teil des Kondensates bestehend aus Kühlmittel, kondensierten Brüden und suspendierten Organika unter Rückkühlung im Kreis führt, den restlichen Teil des Kondensates einer Phasentrennung zuführt, das abgetrennte Wasser einer Abwasseraufarbeitung zuführt und die abgetrennten Organika weiter verwendet.

Bevorzugt wird der restliche Teil des Kondensates bestehend aus Kühlmittel, kondensierten Brüden und suspendierten Organika vor der Phasentrennung soweit aufgewärmt, daß die Organika flüssig vorliegen, um danach eine Flüssig-Flüssig-Phasentrennung durchzuführen.

Alternativ dazu können die Organika aber auch in fester Form aus dem restlichen Kondensat abgetrennt werden.

Bevorzugt werden als Aromaten Toluol, Benzol, Chlorbenzol und Xylol eingesetzt.

Die Aromaten werden mono- oder dinitriert.

Überraschenderweise bilden die Organika trotz der sehr ungünstigen Erstarrungs- und Kristallisationsbedingungen bei der Kondensation der Brüden keine klebrigen Niederschläge. Statt dessen bilden sich feine, harte Kristallite, die eine stabile Suspension ergeben und die nicht zu Verstopfungen oder Belagbildung führen.

Die Erfindung wird im nachfolgenden Beispiel näher erläutert.

### Beispiel

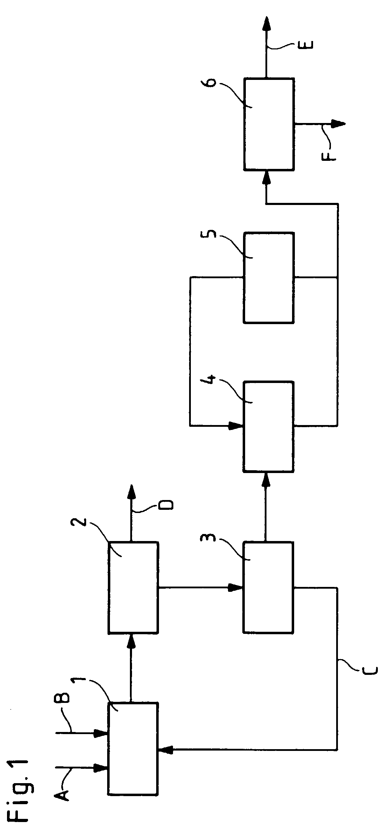

In einem dreistufigen Düsenrohrreaktor 1 (siehe Figur 1) wurden 5,1 kg/h (55,0 mol/h) Toluol A und 12,4 kg/h (118,1 mol/h) 60 gew.-%ige Salpetersäure B gemeinsam mit 209 kg/h Absäure C aus der Säureaufkonzentrierung 3 zur Reaktion gebracht. Das mit 160°C austretende Reaktionsgemisch wurde einer Phasentrennung 2 zugeführt. Es fielen 9,6 kg/h Dinitrotoluol D als Produkt an. Die saure Phase (216 kg/h 80 gew.-%ige Schwefelsäure) wurde in einem Aufkonzentrierschritt 3 auf 82,8 Gew.-% bei einem Druck von 50 mbar aufkonzentriert. Die aufkonzentrierte Absäure C wurde mit 130°C in den Reaktor zurückgeführt.

In einem Kondensator 4 mit einem Volumen von 100 l wurden die 130°C heißen Brüden, bestehend aus 6,6 kg/h Wasserdampf und 0,4 kg/h Dinitrotoluol, kontinuierlich im Sprühstrahl einer Vollkegeldüse unter einem Systemdruck von 50 mbar kondensiert. Die Kühlwassereintrittstemperatur betrug 25°C. Das Dinitrotoluol fiel dabei als kristalliner Feststoff in Wasser suspendiert an. Es kam zu einer Temperaturerhöhung von 5°C. Das aus dem Kondensator 4 abgeführte Kühlwasser-Kondensatgemisch wurde teilweise in einem Wärmeüberträger 5 abgekühlt und erneut als Kühlmedium dem Kondensator 4 zugeführt. Es wurde ein Kreislaufvolumenstrom von 1 m³/h eingestellt. Das restliche Gemisch wurde mit einem Massenstrom von 6,89 kg/h in einem Wärmeüberträger auf 60°C aufgewärmt. Anschließend wurde die organische Phase F in einem Schwerkraftabscheider 6 von der wäßrigen Phase E getrennt (Flüssig/flüssig-Phasen-trennung). Es kam zu keiner Verstopfung an der Düse und zu keinen Ablagerungen in den Rohrleitungen und Armaturen. Eine Wandbelagbildung an den Wärmeübertragungsflächen trat ebenfalls nicht auf.

## Patentansprüche

1. Verfahren zur kontinuierlichen Herstellung von Nitroaromaten durch Nitrierung von Aromaten, indem man den Aromaten in einem Reaktor unter Verwendung einer Nitriersäure, bestehend aus 80 bis 100 Gew.-% an anorganischen Bestandteilen, die sich im wesentlichen aus 60 bis 90 Gew.-% Schwefelsäure, 1 bis 20 Gew.-% Salpetersäure und mindestens 5 Gew.-% Wasser zusammensetzen, und bis zu 20 Gew.-% organischen Bestandteilen, die zu 70 bis 100 Gew.-% aus nitrierten Aromaten und zum Rest aus Nebenprodukten bestehen, unter Einstellung eines Molverhältnisses von Salpetersäure zu Aromat von 0,8 bis 2,5 : 1 in Abhängigkeit von der Nitrierung zur Reaktion bringt, das den Reaktor mit einer Temperatur von mindestens 80°C verlassende Reaktionsgemisch in eine obere Produktphase und eine untere Säurephase auftrennt, die Produktphase aufarbeitet und die mit Organika behaftete Säurephase durch Entspannungsverdampfung gegebenenfalls unter gleichzeitiger Wärmezufuhr von mindestens 5 Gew.-% des Wassers befreit, dadurch gekennzeichnet, daß man nach der Entspannungsverdampfung die Brüden direkt in den Sprühstrahl eines Kühlmittels, vorzugsweise Wasser, im Vakuum einbringt, dabei die Brüden kondensiert, einen Teil des Kondensates bestehend aus Kühlmittel, kondensierten Brüden und suspendierten Organika unter Rückkühlung im Kreis führt, den restlichen Teil des Kondensates einer Phasentrennung zuführt, das abgetrennte Wasser einer Abwasseraufarbeitung zuführt und die abgetrennten Organika weiter verwendet.

2. Verfahren gemäß Anspruch 1, dadurch gekennzeichnet, daß der restliche Teil des Kondensates vor der Phasentrennung soweit aufgewärmt wird, daß die Organika flüssig vorliegen, um danach eine Flüssig-Flüssig-Phasentrennung durchzuführen.

## Claims

1. A process for the continuous production of nitroaromatics by nitration of aromatics, wherein the aromatics are reacted in a reactor with the use of a nitrating acid consisting of 80 to 100 wt.% of inorganic constituents, which are composed substantially of 60 to 90 wt.% sulphuric acid, 1 to 20 wt.% nitric acid and at least 5 wt.% water, and up to 20 wt.% organic constituents consisting to the extent of 70 to 100 wt.% of nitrated aromatics, the remainder being by-products, a molar ratio of nitric acid to aromatic of from 0.8 to 2.5:1, depending on the nitration, being established, the reaction mixture leaving the reactor at a temperature of at least 80°C is separated into an upper product phase and a lower acid phase, the product phase is worked up and the acid phase, which contains organic substances, is freed from at least 5% of the water by flash evaporation optionally with simultaneous input of heat, characterised in that after the flash evaporation, the vapours are introduced under vacuum directly into the directed spray of a cooling agent, preferably water, in the course of this the vapours are condensed, a part of the condensate consisting of cooling agent, condensed vapours and suspended organic substances is recirculated with cooling, the remaining part of the condensate is passed to a phase separation step, the water separated off is passed to a wastewater treatment step and the organic substances separated off are reused.

2. A process according to claim 1, characterised in that before the phase separation step the remaining part of the condensate is heated until the organic substances are in liquid form, in order then to carry out a liquid-liquid phase separation.

## Revendications

1. Procédé pour la préparation en continu de composés nitroaromatiques par nitration de composés aromatiques, dans lequel on amène à réagir les composés aromatiques dans un réacteur en utilisant un mélange sulfonitrique constitué, à concurrence de 80 à 100% en poids, par des constituants inorganiques dont la composition comprend essentiellement, à concurrence de 60 à 90% en poids, de l'acide sulfurique, à concurrence de 1 à 20% en poids, de l'acide nitrique et au moins, à concurrence de 5% en poids, de l'eau, et jusqu'à concurrence de 20% en poids, par des constituants organiques qui sont constitués, jusqu'à concurrence de 70 à 100% en poids, par des composés aromatiques nitrés et, pour le reste, par des sous-produits, en réglant un rapport molaire de l'acide nitrique au composé aromatique de 0,8 à 2,5:1 en fonction de la nitration, on sépare le mélange réactionnel quittant le réacteur avec une température d'au moins 80°C en une phase de produit supérieure et en une phase acide inférieure, on traite la phase de produit et on libère de l'eau à concurrence d'au moins 5% en poids la phase acide contaminée avec des produits organiques par évaporation de détente éventuellement avec apport simultané en chaleur, caractérisé en ce qu'on introduit sous vide, après l'évaporation de détente, les vapeurs directement dans le jet de pulvérisation d'un réfrigérant, de préférence de l'eau, on condense en l'occurrence les vapeurs, on guide en circuit fermé une partie du condensat constitué par le réfrigérant, par les vapeurs condensées et par les composés organiques en suspension par refroidissement en retour, on achemine la partie résiduelle du condensat à une séparation de phases, on achemine l'eau séparée à un traitement des eaux usées et on recycle les composés organiques séparés.

2. Procédé selon la revendication 1, caractérisé en ce qu'on réchauffe la partie résiduelle du condensat avant la séparation de phases jusqu'à ce que les composés organiques soient présents sous forme liquide pour procéder par la suite à une séparation de phases liquide-liquide.
